# EUROPÄISCHE PATENTSCHRIFT

(11) **EP 2 521 572 B1**
(45) Veröffentlichungstag und Bekanntmachung des Hinweises auf die Patenterteilung: **18.12.2013**
(21) Anmeldenummer: 10796026.2
(22) Anmeldetag: 20.12.2010
(51) Int. Cl.: A61L 2/26, C12M 1/34, C12Q 1/00, A61L 2/28

(54) **VERFAHREN UND VORRICHTUNG ZUR MESSUNG DER ABTÖTENDEN WIRKSAMKEIT EINES ENTKEIMUNGSMITTELS**
METHOD AND APPARATUS FOR MEASURING THE KILLING EFFECTIVENESS OF A DISINFECTANT
PROCÉDÉ ET DISPOSITIF DE MESURE DE L'EFFICACITÉ DE DESTRUCTION D'UN DÉSINFECTANT

(30) Priorität: 04.01.2010 DE 102010004001
(43) Veröffentlichungstag der Anmeldung: 14.11.2012
(73) Patentinhaber: SIG Technology AG, 8212 Neuhausen am Rheinfall (CH)
(72) Erfinder: REISERT, Steffen, 52428 Jülich (DE); NÄTHER, Niko, 56073 Koblenz (DE); SCHÖNING, Michael, J., 52428 Jülich (DE); FLÖRKE, Rudolf, 52428 Jülich (DE); GEISSLER, Hanno, 47802 Krefeld (DE)
(74) Vertreter: Cohausz & Florack
(86) Internationale Anmeldenummer: PCT/EP2010/070218
(87) Internationale Veröffentlichungsnummer: WO 2011/080135

(56) Entgegenhaltungen:
- EP-A1- 1 319 411
- EP-A1- 1 882 479
- WO-A2-2007/033212
- R. Ammann et al.: "Merkblatt Prüfung von Aseptikanlagen mit Packmittelentkeimungsvorrichtungen auf deren Wirkungsgrad", VDMA , 30. Juli 2002 (2002-07-30), Seiten 1-13, XP002627201, Gefunden im Internet: URL:http://www.vdma.org/wps/wcm/connect/8e ebbf004dd22dd29c60fcd1f693e3d9/FS_06_2002_ deutsch.pdf?MOD=AJPERES&CACHEID=8eebbf004d d22dd29c60fcd1f693e3d9 [gefunden am 2011-03-09]

## Beschreibung

Die vorliegende Erfindung betrifft ein Verfahren zur Messung der abtötenden Wirksamkeit eines Entkeimungsmittels gegenüber Mikroorganismen und deren Sporen (Überdauerungsformen) mit einem in Kontakt mit dem Entkeimungsmittel stehenden Sensorsystem, wobei die gemessenen Signale von einer mit dem Sensorsystem in Verbindung stehenden Recheneinheit gesammelt werden.

Bei der aseptischen Verarbeitung von Gebrauchsgütern, wie beispielsweise Getränken, findet mittels separater Sterilisierung von Verpackungsbehälter und ggf. Produkt eine Abfüllung des Produktes in die sterilisierten und gereinigten Behälter und daran anschließend ein Verschließen der Behälter statt, um die Haltbarkeit der abgefüllten Produkte für einen gewünschten Zeitraum zu gewährleisten. Dazu werden die Behälter und ggf. die verwendeten Ausgießelemente mittels eines meist dampfförmigen Desinfektionsmittels in Kontakt gebracht, um die darin bzw. darauf befindlichen Keime zuverlässig abzutöten. Als Desinfektionsmittel wird beispielsweise Wasserstoffperoxid (H₂O₂) verwendet. Anschließend werden die Behälter mit - oft heißer - Sterilluft ausgespült, um die Rückstände des Desinfektionsmittels zu entfernen. Dieses bekannte Verfahren ist äußerst effektiv und seit langem bekannt.

Verfahren zur Konzentrationsmessung eines Entkeimungsmediums sind seit langem in unterschiedlichsten Ausführungen bekannt.

Auch ist bekannt, eine bestimmte Anzahl von Testmikroorganismen mit bekannter Resistenz einer Behandlung mit einem Entkeimungsmittel zu unterziehen, um dann anhand der Reduktion dieser Mikroorganismen auf die Entkeimungskraft des getesteten Verfahrens schließen zu können (DE 602 06 254 T2).

Das Merkblatt "Prüfung von Aseptikanlagen mit Packmittelentkeimvorrichtungen auf deren Wirkungsgrad" vom Verband Deutscher Maschinen und Anlagenbau e.V. - Fachverband Nahrungsmittelmaschinen und Verpackungsmaschinen vom Juli 2002 offenbart einen Keimreduktionstest, bei dem mit einem Testkeim künstlich verkeimte Packmittel eine Aseptikanlage durchlaufen. Dabei wird die Anzahl der lebensfähigen Sporen vor und nach dem Passieren der Entkeimungsvorrichtung bestimmt und aus der Differenz der Keimzahlen die Abtötrate ermittelt.

Es ist auch bereits bekannt (DE 600 21 387 T2), Temperatur, Konzentration oder Feuchte des Sterilisationsmittels und auch die Dauer der Behandlung zu messen, um daraus Aussagen über die Sterilisationswirkung des Verfahrens oder des eingesetzten Sterilisationsmittels machen zu können.

Ferner ist eine Anlage bekannt (EP 01 882 479 A1), welche Sensoren nutzt, um zu kontrollieren, ob voreingestellte Zustände des Sterilisationsmittels erreicht und über einen bestimmten Zeitraum gehalten werden. Sind diese Einstellungen erreicht worden, erfolgt der Wechsel in den nächsten Zustand. Weicht die Anlage vom Zustand ab, gibt sie eine Fehlermeldung aus.

Die WO 2007/033212 A2 beschreibt eine Anlage mit mindestens einem Sensor zum Detektieren von Ozon und mindestens einem zum Detektieren von UV-Strahlung. Die Ozon-Sensoren und die UV-Sensoren dienen zum Feststellen und gegebenenfalls speichern der gemessenen Werte. Zusätzlich können Temperatursensoren, Feuchtigkeitssensoren, Gassensoren oder Beschleunigungssensoren vorgesehen sein.

Die vorgenannten Verfahren sind jedoch relativ ungenau, da die Wirkung von vielen Faktoren, welche direkt oder nicht direkt messbar sind, abhängt. Aus diesem Grunde ist stets eine mikrobiologische Bestimmung notwendig, um eine belastbare Aussage zu erhalten. Die biologische Bestimmung ist jedoch nur genau für diesen Sterilisationsvorgang mit den dabei herrschenden Parametern gültig und darüber hinaus äußerst zeitaufwändig.

Der Erfindung liegt daher die Aufgabe zu Grunde, das eingangs genannte und zuvor näher beschriebene Verfahren und eine entsprechende Vorrichtung zur Messung der abtötenden Wirksamkeit eines Entkeimungsmittels so auszugestalten und weiterzubilden, dass die jeweilige Sterilisationswirkung beliebiger Entkeimungsvorgänge und Entkeimungsmittel zuverlässig bestimmt werden kann.

Gelöst wird die vorgenannte Aufgabe bei einem Verfahren gemäß dem Oberbegriff von Patentanspruch 1 dadurch, dass während der Signalerfassung ein validierter Keimreduktionstest zur Bestimmung der Keimreduktionsrate durchgeführt wird und dass die von wenigstens zwei Sensoren des Sensorsystems gemessenen Signale in ein bestimmtes Verhältnis zueinander gesetzt werden und daraus zu jedem Zustand des Entkeimungsmittels ein bestimmtes Muster errechnet wird, welches als Basis für ein reproduzierbares Verhältnis von Signaldaten und Sterilisationswirkung dient und das zur unmittelbaren Bestimmung der jeweiligen Sterilisationswirkung durch Gegenüberstellung des aus den sensorischen Messungen erhaltenen Muster und dem Ergebnis des Keimreduktionstests dient.

Das erfindungsgemäße Verfahren verwendet also eine Mehrzahl von Sensoren, welche aus den relevanten Parametern wie Konzentration des verwendeten Entkeimungsmittels, Temperatur, Luftfeuchte, Volumenstrom, Druck, oxidierenden bzw. reduzierenden Gase bzw. Gasbestandteile etc. ein Summensignal berechnen. Die Parameter betreffen dabei direkte (Temperatur, Druck, Konzentration, etc.) und indirekte (oxidierende und reduzierende Bestandteile, beispielsweise infolge Zerfall von H₂O₂) Größen. Die Gesamtheit der Messsignale der verwendeten Sensoren, die eine Empfindlichkeit gegenüber den relevanten Parametern aufweisen, dient dabei dazu, unterschiedliche "Zustände" des Entkeimungsmittels zu erfassen. Dabei erhält man für jeden Zustand des Entkeimungsmittels ein bestimmtes "Muster" aus den Signalen der Sensoren. Erfindungsgemäß findet zeitgleich zu der Erfassung der Sensorsignale die Durchführung eines validierten Keimreduktionstestes zur Bestimmung der Sterilisationswirkung statt.

Das Ergebnis des Keimreduktionstests, beispielsweise die Keimreduktionsrate, wird dann dem aus den sensorischen Messungen erhaltenen Muster gegenübergestellt. Auf diese Weise wird für beliebige Zustände des Entkeimungsmittels anhand einer "Mustererkennung" ein Verhältnis zwischen Sensordaten und Sterilisationswirkung aufgestellt, wobei nicht zwingend ein lineares mathematisches Verhältnis zwischen diesen beiden Größen bestehen muss. Auf Basis der hinterlegten Daten lässt sich durch eine entsprechende "inverse Mustererkennung" für beliebige, unbekannte Zustände des Entkeimungsmittels, die Sterilisationswirkung anhand der sensorischen Signale bestimmen und über ein Display darstellen.

Das erfindungsgemäße Verfahren unterscheidet sich daher von den bisherigen Ansätzen zur Bestimmung der Sterilisationswirkung mittels Sensoren, da nicht nur die Konzentration des Entkeimungsmittels oder anderer Einflussgrößen wie Luftfeuchte, Temperatur etc. anhand von parametrischen Messgrößen bestimmt werden, sondern anhand der beschriebenen Korrelation die jeweilige Sterilisationswirkung unmittelbar bestimmt wird. Das erfindungsgemäße Verfahren ist daher in der Lage, sämtliche die Sterilisation beeinflussende Größen berücksichtigen zu können. Insofern nutzt das erfinderische Verfahren die Kenntnis aus, dass sich je nach verwendetem Entkeimungsmittel die keimtötende Wirkung nicht allein aus der Konzentration des Entkeimungsmittels, der Temperatur oder anderen Parametern ergibt. Beispielsweise kann die keimtötende Wirkung eines Entkeimungsmittels durch die oxidativen Eigenschaften chemischer Verbindungen begünstig werden, welche unter gewissen Voraussetzungen (Temperatur, Druck, Feuchte etc.) aus dem Entkeimungsmittel hervorgehen.

Gemäß einer weiteren Lehre der Erfindung wird ein Sensorsystem verwendet, welches in unmittelbarem Kontakt mit dem verwendeten dampfartigen oder gasförmigen Entkeimungsmittel steht. Alternativ ist es jedoch auch möglich, dass bei einem flüssigen Entkeimungsmittel das Sensorsystem in mittelbarem Kontakt mit dem Entkeimungsmittel steht, also oberhalb dessen Pegel angeordnet ist. Als Sensorsystem kann eine für sich bekannte Multisensoranordnung, häufig auch als "künstliche Nase" bezeichnet zum Einsatz kommen. Solche Multisensoranordnungen weisen eine Vielzahl von Sensoren auf und sind seit längerem bekannt (DE 102 36 327 A1).

Eine weitere Ausführung der Erfindung sieht vor, dass die Erfassung und Gegenüberstellung der sensorischen Signale und mikrobiologischen Messungen zur Kalibrierung des Sensorsystems dienen. Auf diese Weise lässt sich auf Basis der hinterlegten Daten durch eine entsprechende inverse Mustererkennung für beliebige, unbekannte Zustände des Entkeimungsmittels die Sterilisationswirkung anhand der Sensorsignale bestimmen.

In weiterer Ausgestaltung der Erfindung wird durch Kopplung der sensorischen Signale während eines Entkeimungsvorgangs kontinuierlich ein Signal erzeugt, welches proportional zur Wirksamkeit des Entkeimungsprozesses ist. Dieses erzeugte Signal kann entweder offline oder online verarbeitet werden.

Darüber hinaus ist erfindungsgemäß vorgesehen, dass als Entkeimungsmittel Wasserstoffperoxid, Peressigsäure, Ethanol oder andere bekannte Substanzen einsetzbar sind. Das verwendete Sensorsystem kann also mit beliebigen Entkeimungsmitteln kalibriert und angewandt werden.

Vorrichtungsmäßig wird die Aufgabe dadurch gelöst, dass das Sensorsystem eine Mehrzahl von Sensoren aufweist, die zu einer Multisensoreinheit zusammengefasst sind, welche mit einer Recheneinheit in Verbindung steht und wobei die Vorrichtung Mittel zur Ausführung des Verfahrens nach den Ansprüchen 1 bis 9 aufweist.

Die Sensoren des Sensorsystems können dabei in dampfartigen oder gasförmigen Medien bei Temperaturen bis zu 900° C eingesetzt werden.

Nach einer weiteren Lehre der Erfindung umfasst das Mittel zur Ausführung mindestens eines der folgenden Elemente: eine Multisensoreinheit, eine Recheneinheit, einen Datenaufnehmer, ein Steuergerät, einen Messumformer, einen Speicher, ein Display, einen externen Bildschirm, einen Drucker und/oder ein Netzgerät.

Gemäß einer weiteren Lehre der Erfindung weist das Sensorsystem elektrische Sensoren auf. Hier kommen beispielsweise handelsübliche Halbleitergassensoren zum Einsatz. In weiterer Ausbildung der Erfindung kann das Sensorsystem auch/und elektrochemische Sensoren wie beispielsweise Festkörperelektrolytgassensoren enthalten. Alternativ ist es auch möglich, dass das Sensorsystem kalorimetrische Gassensoren aufweist, welche mit einem Katalysator verbunden sind, beispielsweise mit Pellistoren. Es ist klar, dass mit der Anzahl der verwendeten Sensoren die Genauigkeit des erfindungsgemäßen Verfahrens steigt.

Gemäß einer weiteren bevorzugten Ausgestaltung der Erfindung weist die Recheneinheit einen Datenaufnehmer zur Datenübertragung auf, der über einen Messumformer mit einer Recheneinheit in Verbindung steht. Bevorzugt sorgt ein Speicher für die Speicherung von Ausgangsgrößen und/oder Kalibrierungsdaten. Eine weitere Lehre der Erfindung sieht vor, dass ein Steuergerät zur Regelung der Betriebsparameter der verwendeten Sensoren des Sensorensystems dient. Zur Spannungsversorgung der einzelnen Bausteine ist ein geeignetes Netzgerät vorgesehen.

Die Erfindung wird nachfolgend anhand einer lediglich ein bevorzugtes Ausführungsbeispiel darstellenden Zeichnung näher erläutert. In der Zeichnung zeigen
- Fig. 1: eine erfindungsgemäße Vorrichtung, schematisch als Blockschaltbild,
- Fig. 2a, 2b und 2c: verschiedene Positionierungsmöglichkeiten des Sensorsystems und
- Fig. 3: eine Gegenüberstellung der Messwerte zweier Sensoren.

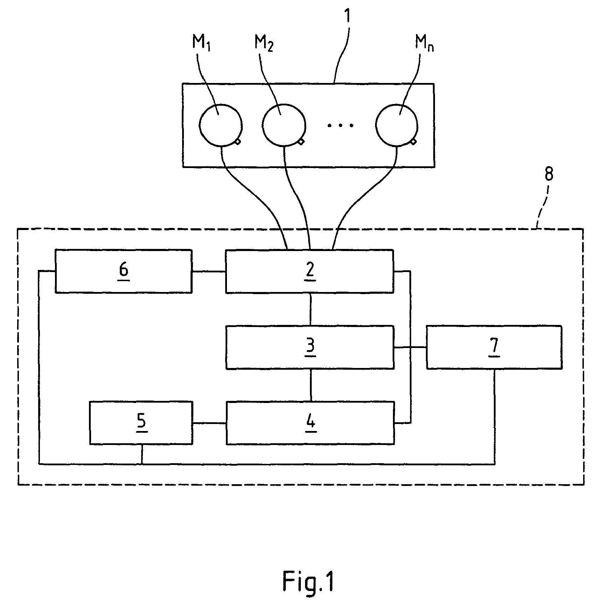

In Fig. 1 ist schematisch der Aufbau einer erfindungsgemäßen Vorrichtung zur Durchführung des erfindungsgemäßen Verfahrens dargestellt. Dabei ist mit 1 eine Multisensoranordnung gezeigt, in der eine Mehrzahl einzelner Sensoren M₁, M₂, ..., Mₙ angeordnet ist. Die Multisensoranordnung 1 kann wahlweise aus einem Edelmetall, hitze- und/oder säurebeständigem Kunststoff oder einem anderen geeigneten Material bestehen.

Die einzelnen Sensoren M₁ bis Mₙ der Multisensoranordnung 1 sind über nicht näher bezeichnete Kabelleitungen mit einem Datenaufnehmer 2 verbunden. Dadurch kann die Multisensoranordnung 1 unabhängig von der Position des Datenaufnehmers 2 an einem beliebigen Ort angebracht werden. Der Datenaufnehmer 2 dient der Datenübertragung zwischen den n Sensoren M₁ bis Mₙ der Multisenoreinheit 1, einem nachgeschalteten Messumformer 3 und einem Steuergerät 6. Über das Steuergerät 6 werden die Betriebsparameter der n Sensoren M₁ bis Mₙ geregelt. Diese umfassen beispielsweise die Betriebstemperatur, die Versorgungsspannung oder den Messstrom. Der Messumformer 3 dient dabei der Wandlung der analogen Sensorsignale in digitale Daten. Diese werden von der Recheneinheit 4 verarbeitet.

Neben der Recheneinheit 4 erkennt man einen Speicher 5, in dem die Daten der Ausgangsgrößen bzw. der Kalibrierungsdaten hinterlegt sind. Auf Basis eines Vergleichs der gemessenen Daten mit den im Speicher 5 hinterlegten Kalibrierungsdaten erfolgt in der Recheneinheit 4 simultan die Berechnung der jeweiligen Ausgangsgröße. Die Ausgangsgröße kann wahlweise über ein in der Recheneinheit 8 integriertes Display, einen externen Bildschirm oder einen Drucker (alle drei nicht dargestellt) ausgegeben und/oder im Speicher 5 abgelegt werden.

Ein Netzgerät 7 stellt die Versorgungsspannung für die Komponenten 2 bis 6 sicher. Wahlweise können die Komponenten 2 bis 7 ganz oder teilweise in einem Gehäuse untergebracht sein. Es ist ebenfalls denkbar, dass alle oder Teile der Komponenten 2 bis 7 durch einen PC ersetzt bzw. ergänzt werden. Die beschriebene Messeinrichtung kann beispielsweise als fester Bestandteil in einem Sterilisierapparat integriert sein.

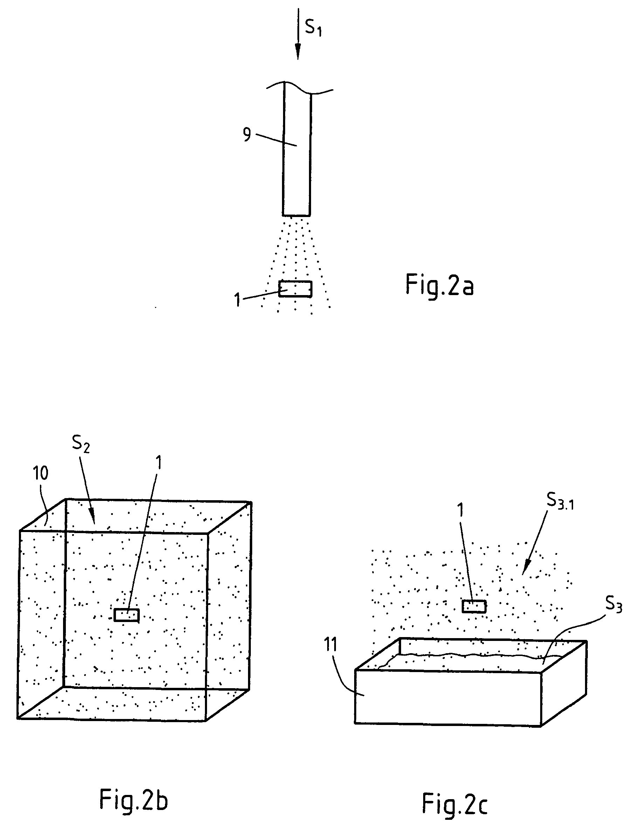

Fig. 2 zeigt mögliche Anordnungen der Multisensoranordnung 1 in einem Sterilisierapparat. Die Multisensoranordnung 1 kann wahlweise unmittelbar in der Nähe des Auslasses einer Zuführeinrichtung 9 für ein dampfartiges bzw. gasförmiges Sterilisationsmittel S₁ (siehe Fig. 2a), an einem beliebigen Ort in einer Sterilisationskammer 10, bei der das dampfartige bzw. gasförmige Sterilisationsmittel S₂ im Raum verteilt ist (siehe Fig. 2b) oder im Falle eines flüssigen Sterilisationsmittels S₃ über dem Flüssigkeitsspiegel angeordnet sein, wobei ein Teil des Sterilisationsmittels in gasförmigem Aggregatszustand S_{3.1} in Kontakt mit der Multisensoranordnung 1 steht (siehe Fig. 2c).

Nicht dargestellt ist, dass die erfindungsgemäße Messeinrichtung auch als Kompaktsystem im Sinne eines tragbaren Handgerätes ausgeführt sein kann, wodurch ortsveränderliche Messungen ermöglicht werden. Dabei kann die Übertragung von Daten zwischen den einzelnen Komponenten auch drahtlos erfolgen.

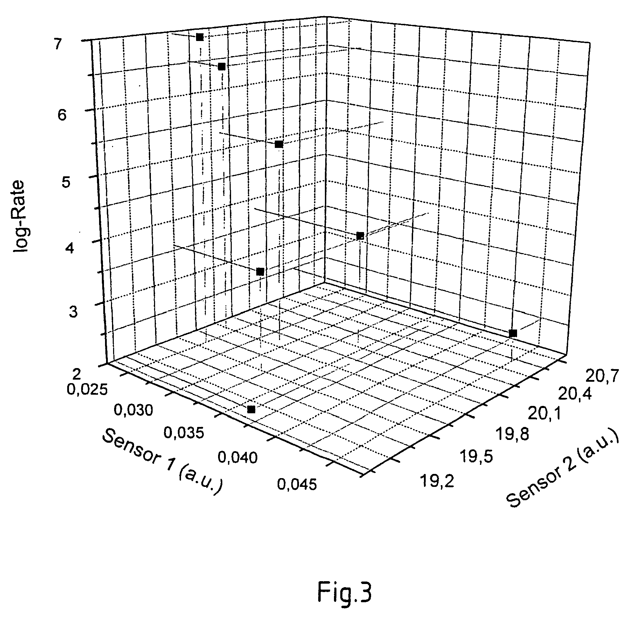

Fig. 3 zeigt schließlich eine Gegenüberstellung der Messwerte zweier Sensoren Sensor 1 und Sensor 2 als willkürliche Einheiten (sog. "a.u. = arbitrary units") mit den Ergebnissen eines Keimreduktionstests in Form gängiger log-Raten. Die log-Raten geben die Reduktionsrate eines Testkeims in Zehnerpotenzen an.

Im dargestellten und insoweit bevorzugten Ausführungsbeispiel gemäß Fig. 3 sind sieben nicht näher bezeichnete Messpunkte dargestellt, welche für verschiedene "Zustände" eines Entkeimungsmittels aufgezeichnet wurden. Die Erfassung und Gegenüberstellung dieser Daten für verschiedene "Zustände" des Entkeimungsmittels entspricht der Kalibrierung des Sensorsystems. Die Mustererkennung, welche die erfassten Daten in ein mathematisches Verhältnis setzt, sowie die "inverse Mustererkennung", welche aus den sensorischen Messwerten eines unbekannten "Zustandes" den entsprechenden Wirkungsgrad errechnet, sind nicht dargestellt.

Theoretisch lässt sich das erfindungsgemäße Verfahren auch mit einem einzigen Sensor einsetzen, auch wenn im bevorzugten Ausführungsbeispiel die Verwendung zweier Sensoren beschrieben ist. Zur Erzielung einer hinreichend genauen Messung der abtötenden Wirksamkeit eines Entkeimungsmittels sollten jedoch mindestens zwei Sensoren verwendet werden, wobei sich die Genauigkeit des Messverfahrens mit zunehmender Anzahl der Sensoren verbessern kann.

Das erfindungsgemäße Verfahren bzw. die erfindungsgemäße Vorrichtung erlaubt es, sehr viele Parameter zu variieren und zeitnah eine Aussage über die Wirksamkeit zu erhalten. Zudem kann das Verfahren so gezielt verändert werden, um bestimmte Wirkungsgrade zu erreichen.

## Patentansprüche

1. Verfahren zur Messung der abtötenden Wirksamkeit eines Entkeimungsmittels gegenüber Mikroorganismen und deren Sporen mit einem in Kontakt mit dem Entkeimungsmittel stehenden Sensorsystem, wobei die gemessenen Signale von einer mit dem Sensorsystem in Verbindung stehenden Recheneinheit gesammelt werden,
**dadurch gekennzeichnet, dass**
während der Signalerfassung ein validierter Keimreduktionstest zur Bestimmung der Keimreduktionsrate durchgeführt wird und dass die von wenigstens zwei Sensoren des Sensorsystems gemessenen Signale in ein bestimmtes Verhältnis zueinander gesetzt werden und daraus zu jedem Zustand des Entkeimungsmittels ein bestimmtes Muster errechnet wird, welches als Basis für ein reproduzierbares Verhältnis von Signaldaten und Sterilisationswirkung dient und das zur unmittelbaren Bestimmung der jeweiligen Sterilisationswirkung durch Gegenüberstellung des aus den sensorischen Messungen erhaltenen Muster und dem Ergebnis des Keimreduktionstests dient.

2. Verfahren nach Anspruch 1,
**dadurch gekennzeichnet, dass**
das Entkeimungsmittel dampfartig oder gasförmig ist und in unmittelbarem Kontakt mit dem Sensorsystem steht.

3. Verfahren nach Anspruch 1,
**dadurch gekennzeichnet, dass**
das Entkeimungsmittel flüssig ist und in mittelbarem Kontakt mit dem Sensorsystem steht.

4. Verfahren nach einem der Ansprüche 1 bis 3,
**dadurch gekennzeichnet, dass**
die Sensoren des Sensorsystems auf die Einflussparameter des Sterilisationsverfahrens und deren Veränderung reagieren.

5. Verfahren nach einem der Ansprüche 1 bis 4,
**dadurch gekennzeichnet, dass**
die Erfassung und Gegenüberstellung der sensorischen Signale und der mikrobiologischen Messungen zur Kalibrierung des Sensorsystems dienen.

6. Verfahren nach einem der Ansprüche 1 bis 5,
**dadurch gekennzeichnet, dass**
durch Kopplung der sensorischen Signale während eines Entkeimungsvorgangs kontinuierlich ein Signal erzeugt wird, welches eine Abhängigkeit zur Wirksamkeit des Entkeimungsprozesses aufweist.

7. Verfahren nach Anspruch 6,
**dadurch gekennzeichnet, dass**
das erzeugte Signal offline verarbeitet wird.

8. Verfahren nach Anspruch 6,
**dadurch gekennzeichnet, dass**
das erzeugte Signal online verarbeitet wird.

9. Verfahren nach einem der Ansprüche 1 bis 8,
**dadurch gekennzeichnet, dass**
als Entkeimungsmittel Wasserstoffperoxid, Peressigsäure, Ethanol oder andere bekannte Substanzen einsetzbar sind.

10. Vorrichtung zur Durchführung des Verfahrens nach einem der Ansprüche 1 bis 9,
**dadurch gekennzeichnet, dass**
das Sensorsystem eine Mehrzahl von Sensoren (M₁, M₂, ..., Mₙ) aufweist, die zu einer Multisensoreinheit (1) zusammengefasst sind, welche mit einer Recheneinheit (4) in Verbindung steht und wobei die Vorrichtung Mittel zur Ausführung des Verfahrens nach einem der Ansprüche 1 bis 9 aufweist.

11. Vorrichtung nach Anspruch 10,
**dadurch gekennzeichnet, dass**
die Mittel zur Ausführung mindestens eines folgender Elemente umfassen: eine Multisensoreinheit (1), eine Recheneinheit (4), einen Datenaufnehmer (2), ein Steuergerät (6), einen Messumformer (3), einen Speicher (5), ein Display, einen externen Bildschirm, einen Drucker und/oder ein Netzgerät (7).

12. Vorrichtung nach Anspruch 10 oder 11,
**dadurch gekennzeichnet, dass**
das Sensorsystem elektrische Sensoren verwendet.

13. Vorrichtung nach einem der Ansprüche 10 bis 12,
**dadurch gekennzeichnet, dass**
das Sensorsystem elektrochemische Sensoren verwendet.

14. Vorrichtung nach einem der Ansprüche 10 bis 13,
**dadurch gekennzeichnet, dass**
das Sensorsystem kalorimetrische Sensoren verwendet.

15. Vorrichtung nach einem der Ansprüche 10 bis 13,
**dadurch gekennzeichnet, dass**
die Recheneinheit (4) einen Datenaufnehmer (2) zur Datenübertragung aufweist, der über einen Messumformer (3) mit einer Recheneinheit (4) in Verbindung steht.

16. Vorrichtung nach Anspruch 15,
**gekennzeichnet durch** einen Speicher (5)
zur Speicherung von Ausgangsgrößen und/oder Kalibrierungsdaten.

17. Vorrichtung nach Anspruch 14 oder 16,
**gekennzeichnet durch** ein Steuergerät (6) zur Regelung der Betriebsparameter der Sensoren (M₁, M₂, ..., Mₙ).

18. Vorrichtung nach den Ansprüchen 14 bis 17,
**dadurch gekennzeichnet, dass**
zur Spannungsversorgung von Datenaufnehmer (2), Messumformer (3), Recheneinheit (4), Speicher (5) und Steuergerät (6) ein Netzgerät (7) vorgesehen ist.

## Claims

1. Method for measuring the killing effectiveness of a disinfectant for microorganisms and the spores thereof using a sensor system in contact with the disinfectant, wherein the measured signals are collected by a computer unit in communication with the sensor system, **characterised in that** a validated bacteria reduction test to determine the bacteria reduction rate is performed while the signals are being collected and that the signals measured by at least two sensor system sensors are placed in a specific relation to one another and a specific pattern for each state of the disinfectant is calculated therefrom, which is used as a basis for a reproducible ratio between signal data and sterilisation effect and for immediate identification of the respective sterilisation effect by comparing the pattern obtained from the sensor measurements and the result of the bacteria reduction test.

2. Method according to claim 1, **characterised in that** the disinfectant is vaporous or gaseous and is in direct contact with the sensor system.

3. Method according to claim 1, **characterised in that** the disinfectant is a liquid and is in direct contact with the sensor system.

4. Method according to any one of claims 1 to 3, **characterised in that** the sensor system sensors respond to the influencing factors of the sterilisation method and any changes thereto.

5. Method according to any one of claims 1 to 4, **characterised in that** the capture and comparison of the sensor signals and the microbiological measurements are used to calibrate the sensor system.

6. Method according to any one of claims 1 to 5, **characterised in that** a signal is generated continually by linking the sensor signals during a disinfection procedure, which signal is dependent upon the effectiveness of the disinfectant procedure.

7. Method according to claim 6, **characterised in that** the generated signal is processed offline.

8. Method according to claim 6, **characterised in that** the generated signal is processed online.

9. Method according to any one of claims 1 to 8, **characterised in that** hydrogen peroxide, peracetic acid, ethanol or other known substances can be used as disinfectants.

10. Apparatus for carrying out the method according to any one of claims 1 to 9, **characterised in that** the sensor system has a plurality of sensors (M₁, M₂,..., Mₙ), which are combined into a multi-sensor unit (1), which is connected to a computer unit (4) and wherein the apparatus has means to implement the method according to any one of claims 1 to 9.

11. Apparatus according to claim 10, **characterised in that** the implementation means comprises at least one of the following elements: a multi-sensor unit (1), a computer unit (4), a data recorder (2), a control device (6), a transducer (3), a memory (5), a display, an external monitor, a printer and/or a network device (7).

12. Apparatus according to claim 10 or 11, **characterised in that** the sensor system uses electrical sensors.

13. Apparatus according to any one of claims 10 to 12, **characterised in that** the sensor system uses electrochemical sensors.

14. Apparatus according to any one of claims 10 to 13 **characterised in that** the sensor system uses calorimetric sensors.

15. Apparatus according to any one of claims 10 to 13, **characterised in that** the computer unit (4) has a data recorder (2) for data transmission which is connected to a computer unit (4) via a transducer (3).

16. Apparatus according to claim 15, **characterised by** a memory (5) for storing output variables and/or calibration data.

17. Apparatus according to claim 14 or 16, **characterised by** a control device (6) for controlling the operating parameters of the sensors (M₁, M₂, ..., Mₙ).

18. Apparatus according to claims 14 to 17, **characterised in that** a network device (7) is provided to supply power to the data recorder (2), transducer (3), computer unit (4), memory (5) and control device (6).

## Revendications

1. Procédé de mesure de l'efficacité germicide d'un agent de désinfection en présence de microorganismes et de leurs spores, avec un système de détection, qui est en contact avec ledit agent de désinfection, sachant que les signaux mesurés sont collectés par une unité de calcul qui est en relation avec le système de détection,
**caractérisé en ce que**,
pendant la saisie des signaux, un test validé, relatif à la réduction des germes, est effectué en vue de la détermination des taux de réduction des germes, et que le rapport est établi entre les signaux, qui sont mesurés par au moins deux détecteurs du système de détection, et qu'un modèle déterminé est calculé sur cette base, pour chaque état de l'agent de désinfection, ledit modèle servant de base à un rapport reproductible de données de signaux et d'effet de stérilisation, ainsi qu'à la détermination directe de chaque effet de stérilisation par comparaison avec le modèle obtenu à partir des mesures sensorielles, et au résultat du test de réduction des germes.

2. Procédé selon la revendication 1,
**caractérisé en ce que**
l'agent de désinfection est en forme de vapeur ou de gaz et qu'il est en contact direct avec le système de détection.

3. Procédé selon la revendication 1,
**caractérisé en ce que**
l'agent de désinfection est liquide et est en contact indirect avec le système de détection.

4. Procédé selon l'une des revendications 1 à 3,
**caractérisé en ce que**
les détecteurs du système de détection réagissent aux paramètres d'influence du procédé de désinfection et à leurs modifications.

5. Procédé selon l'une des revendications 1 à 4,
**caractérisé en ce que**
la saisie et la comparaison des signaux de détection et des mesures microbiologiques servent au calibrage du système de détection.

6. Procédé selon l'une des revendications 1 à 5,
**caractérisé en ce que**,
par couplage des signaux de détection, pendant le processus de désinfection, est continuellement généré un signal, qui est en dépendance de l'efficacité du processus de désinfection.

7. Procédé selon la revendication 6
**caractérisé en ce que**
le signal généré est traité offline.

8. Procédé selon la revendication 6,
**caractérisé en ce que**
le signal généré est traité online.

9. Procédé selon l'une des revendications 1 à 8,
**caractérisé en ce que**,
comme agent de désinfection, on peut utiliser du peroxyde d'hydrogène, de l'acide per-acétique, de l'éthanol ou d'autres substances connues.

10. Dispositif pour la réalisation du procédé selon l'une des revendications 1 à 9,
**caractérisé en ce que**
le système de détection est doté d'une pluralité de détecteurs (M₁, M₂, ..., Mₙ), qui sont groupés en une unité multi-détecteurs (1), laquelle est en liaison avec une unité de calcul (4), et sachant que le dispositif est doté de moyens d'exécution du procédé selon l'une des revendications 1 à 9.

11. Dispositif selon la revendication 10,
**caractérisé en ce que**
les moyens d'exécution comprennent au moins l'un des éléments suivants : une unité multi-détecteurs (1), une unité de calcul (4), un collecteur de données (2), un appareil de commande (6), un convertisseur de mesure (3), une mémoire (5), un display, un écran de visualisation externe, une imprimante et / ou un bloc d'alimentation secteur (7).

12. Dispositif selon revendication 10 ou 11,
**caractérisé en ce que**
des détecteurs électriques sont utilisés pour le système de détection.

13. Dispositif selon l'une des revendications 10 à 12,
**caractérisé en ce que**
des détecteurs électrochimiques sont utilisés pour le système de détection.

14. Procédé selon l'une des revendications 10 à 13,
**caractérisé en ce que**
des détecteurs calorimétriques sont utilisés pour le système de détection.

15. Procédé selon l'une des revendications 10 à 13,
**caractérisé en ce que**,
pour la transmission des données, l'unité de calcul (4) est dotée d'un collecteur de données (2), qui est en liaison avec une unité de calcul (4), par l'intermédiaire d'un convertisseur de mesure (3).

16. Procédé selon la revendication 15,
**caractérisé par**
une mémoire (5) pour la mémorisation de grandeurs de sortie et / ou de données de calibrage.

17. Procédé selon revendication 14 ou 16,
**caractérisé par**
un appareil de commande (6) pour la régulation des paramètres de service des détecteurs (M₁, M₂, ..., Mₙ).

18. Procédé selon l'une des revendications 14 à 17,
**caractérisé en ce que**,
pour l'alimentation en tension du collecteur de données (2), du convertisseur de mesure (3), de l'unité de calcul (4), de la mémoire (5) et de l'appareil de commande (6), est prévu un bloc secteur (7).
